# EUROPÄISCHE PATENTSCHRIFT

(11) **EP 3 265 451 B1**
(45) Veröffentlichungstag und Bekanntmachung des Hinweises auf die Patenterteilung: **26.08.2020**
(21) Anmeldenummer: 16707808.8
(22) Anmeldetag: 04.03.2016
(51) Int. Cl.: C07D 309/12

(54) **VERFAHREN ZUR HERSTELLUNG VON TETRAHYDROPYRANYLESTERN**
METHOD FOR THE PREPARATION OF TETRAHYDROPYRANYL ESTERS
PROCÉDÉ DE FABRICATION D'ESTERS DE TÉTRAHYDROPYRANE

(30) Priorität: 05.03.2015 EP 15157835
(43) Veröffentlichungstag der Anmeldung: 10.01.2018
(60) Teilanmeldung: 18206125.9
(73) Patentinhaber: BASF SE, 67056 Ludwigshafen am Rhein (DE)
(72) Erfinder: RUEDENAUER, Stefan, 69469 Weinheim (DE); STORK, Timon, 68642 Buerstadt Bobstadt (DE); PELZER, Ralf, 37699 Fuerstenberg (DE); HICKMANN, Volker, 67063 Ludwigshafen (DE); KLOS, Margarethe, 67240 Bobenheim Roxheim (DE)
(86) Internationale Anmeldenummer: PCT/EP2016/054632
(87) Internationale Veröffentlichungsnummer: WO 2016/139338

(56) Entgegenhaltungen:
- EP-A1- 0 949 239
- WO-A1-2009/130192
- KARUNA SHANKER PANDEY ET AL: "Synthesis and Bioevaluation of Alicyclic and Heterocyclic Alkanoates as Cockroach Attractants", BIOSCIENCE, BIOTECHNOLOGY, AND BIOCHEMISTRY, Bd. 59, Nr. 4, 1. Januar 1995 (1995-01-01) , Seiten 725-727, XP055188243, ISSN: 0916-8451, DOI: 10.1271/bbb.59.725 in der Anmeldung erwähnt

## Beschreibung

### HINTERGRUND DER ERFINDUNG

Die vorliegende Erfindung betrifft ein Verfahren zur Herstellung von Tetrahydropyranylestern aus den entsprechenden 4-Hydroxy-tetrahydropyranverbindungen durch Umsetzung mit einer Ketenverbindung.

### STAND DER TECHNIK

Zur Herstellung von Konsum- und Verbrauchsgütern mit bestimmten organoleptischen Eigenschaften, d. h. von Produkten, die über vorteilhafte geruchliche (olfaktorische) oder geschmackliche (gustatorische) Eigenschaften verfügen, steht eine große Vielzahl an Aromachemikalien (Riech- und Geschmacksstoffen) für die äußerst diversen Einsatzbereiche dieser Stoffe zur Verfügung. Hier besteht ein ständiger Bedarf an neuen besseren Herstellungsverfahren, die die Bereitstellung einzelner Aromachemikalien z. B. mit höherer Effizienz oder in höherer Reinheit ermöglichen. Diese Herstellungsverfahren sollen auch geeignet sein, die Produkte in größeren, speziell technischen, Maßstäben herzustellen.

Es ist bekannt, zur Herstellung von Estern höherer Alkohole diese mit Carbonsäurehalogeniden oder mit Carbonsäureanhydriden umzusetzen. Nachteilig bei der Umsetzung mit Carbonsäurehalogeniden ist, dass bei deren Umsetzung Halogenwasserstoffsäuren gebildet werden, die allgemein zu Korrosionsproblemen führen und bei tertiären Alkoholen Wasserabspaltungen und dadurch vielfach Polymerisationen verursachen. Bei der Umsetzung mit Carbonsäureanhydriden besteht der Nachteil, dass im Reaktionsgemisch äquimolare Mengen der entsprechenden Carbonsäure gebildet werden, die bei der Aufarbeitung abgetrennt werden müssen und deren Wiederverwendung technisch aufwändig sein kann.

Pandey, et al beschreiben in Biosci. Biotech. Biochem. 59 (4) S. 725-727, 1995 u.a. die Herstellung von 4-Alkanoyltetrahydropyranestern durch Umsetzung des korrespondierenden Alkohols mit Akanoylchlorid und Triethylamin.

Die WO 2009/130192 A1 beschreibt u.a. die Acetylierung von Tetrahydropyranylestern mit Säureanhydriden und Säurechloriden.

Es ist weiterhin bekannt, zur Herstellung von Essigsäureestern hydroxylgruppenhaltige Verbindungen mit Keten umzusetzen. Für die Umsetzung hydroxylgruppenhaltiger

Verbindungen mit Keten können verschiedene Katalysatoren eingesetzt werden, z. B. Brönstedtsäuren, wie Schwefelsäure, p-Toluolsulfonsäure, Phosphorsäure, Kaliumhydrogensulfat oder Lewissäuren, wie Bortrifluorid bzw. Bortrifluorid-Ätherat. Jedoch sind auch für die katalysierte Umsetzung von Ketenen verschiedene Nachteile beschrieben. So können saure Katalysatoren in Metallapparaturen Korrosion hervorrufen oder zur unerwünschten Bildung von harzartigen Verunreinigungen führen. Zudem lassen sie sich vielfach nur schwer wieder vom Reaktionsgemisch abtrennen.

Verfahren und Vorrichtungen zur Herstellung von Keten sind z. B. in Organic Syntheses, Coll. Vol. 1, S. 330 (1941) und Vol. 4, S. 39 (1925) sowie in der Chemiker Zeitung 97, Nr. 2, Seiten 67 bis 73 (1979) beschrieben.

Die EP 0 949 239 A1 beschreibt ein Verfahren zur Herstellung von Linalylacetat durch Umsetzung von Linalool mit Keten in Gegenwart eines Zinksalzes als Katalysator.

Es ist bekannt, diverse substituierte Tetrahydropyranverbindungen als Aromachemikalien zu verwenden. So sind beispielsweise 2,4,4-substituierte Tetrahydropyranylester der allgemeinen Formel (A) wertvolle Aromachemikalien:

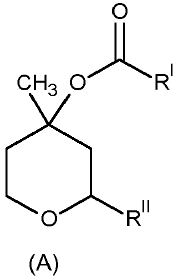

Die EP 0383446 A2 beschreibt die Synthese sowie die olfaktorischen Eigenschaften einer Vielzahl verschiedener 2,4,4-trisubstituierter Tetrahydropyranylester (A), worin R^{I} für Methyl oder Ethyl steht und R^{II} für geradkettiges oder verzweigtes C₂-C₄-Alkyl oder C₂-C₄-Alkenyl steht. Dazu wird zunächst 3-Methylbut-3-en-1-ol mit einem Aldehyd der Formel R^{II}-CHO in Gegenwart eines sauren Katalysators umsetzt, wobei ein Reaktionsgemisch erhalten wird, das wenigstens ein 2-substituiertes 4-Hydroxy-4-methyl-tetrahydropyran der allgemeinen Formel (B) enthält:

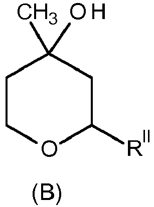

Die Zwischenstufe (B) wird anschließend einer Acylierung durch Umsetzung mit einem Carbonsäureanhydrid unter sauren Bedingungen unterzogen.

4-Hydroxy-tetrahydropyranverbindungen und speziell 2-substituierte 4-Hydroxy-4-methyl-tetrahydropyrane sind ebenfalls wertvolle Verbindungen für den Einsatz als Aromachemikalien, und dem Fachmann sind diverse Verfahren zu ihrer Herstellung bekannt, z. B. aus der EP 1 493 737 A1, WO 2011/147919, WO 2010/133473, WO 2011/154330 und PCT/EP2013/071409.

Es wurde jetzt überraschenderweise gefunden, dass durch Umsetzung von 4-Hydroxy-tetrahydropyranverbindungen und speziell von 2-substituierten 4-Hydroxy-4-methyl-tetrahydropyranen mit Ketenen die Herstellung von Tetrahydropyranylestern und speziell von 2-substituiertem 4-Hydroxy-4-methyl-tetrahydropyranylestern in einfacher Weise mit sehr hohen Ausbeuten und gleichzeitig in hoher Reinheit gelingt. Somit können bevorzugt Tetrahydropyranylester mit höherer Reinheit und somit besserer Riechstoffqualität erzielt werden als mit aus dem Stand der Technik bekannten Verfahren. Auf aufwändige Reinigungsschritte kann vorteilhafterweise verzichtet werden. Dies ist angesichts der hohen Reaktivität der eingesetzten Ketene überraschend.

Weitere Vorteile des gefundenen Verfahrens sind, dass die Produkte auch ohne Anwesenheit eines externen Lösungsmittels während der Reaktion in guten Ausbeuten und in hohen Reinheiten erhalten werden, sowie dass der Reaktionsverlauf sehr gut gesteuert und die Reaktion somit gut kontrolliert werden kann.

### ZUSAMMENFASSUNG DER ERFINDUNG

Gegenstand der Erfindung ist ein Verfahren zur Herstellung von Tetrahydropyranylestern der allgemeinen Formel (I) 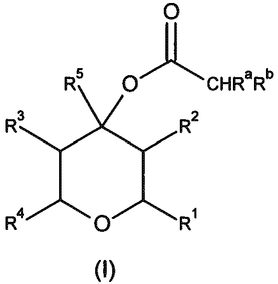 worin
R¹, R², R³ und R⁴ unabhängig voneinander für Wasserstoff, geradkettiges oder verzweigtes C₁-C₁₂-Alkyl, geradkettiges oder verzweigtes C₂-C₁₂-Alkenyl, unsubstituiertes oder mit C₁-C₁₂-Alkyl und/oder C₁-C₁₂-Alkoxy substituiertes Cycloalkyl mit insgesamt 3 bis 20 Kohlenstoffatomen oder unsubstituiertes oder mit C₁-C₁₂-Alkyl und/oder C₁-C₁₂-Alkoxy substituiertes Aryl mit insgesamt 6 bis 20 Kohlenstoffatomen stehen,
R⁵ für Wasserstoff oder geradkettiges oder verzweigtes C₁-C₁₂-Alkyl steht, und
R^{a} und R^{b} für Wasserstoff stehen,
bei dem man wenigstens eine 4-Hydroxy-tetrahydropyranverbindung der allgemeinen Formel (II) bereitstellt 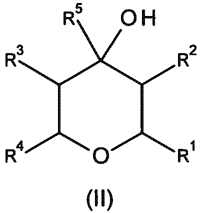
wobei R¹, R², R³, R⁴ und R⁵ die zuvor angegebenen Bedeutungen haben und die Verbindung der allgemeinen Formel (II) einer Umsetzung mit einem Keten (III)

   CR^{a}R^{b}=C=O (III)
unterzieht, wobei R^{a} und R^{b} die zuvor angegebenen Bedeutungen haben.

Eine bevorzugte Ausführungsform ist ein Verfahren zur Herstellung von 2-substituierten 4-Methyltetrahydropyranylestern der allgemeinen Formel (I.1) 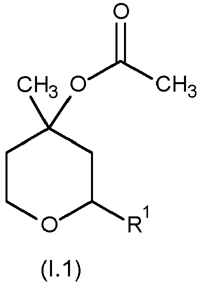 worin
R¹ für Wasserstoff, geradkettiges oder verzweigtes C₁-C₁₂-Alkyl, geradkettiges oder verzweigtes C₂-C₁₂-Alkenyl, unsubstituiertes oder mit C₁-C₁₂-Alkyl und/oder C₁-C₁₂-Alkoxy substituiertes Cycloalkyl mit insgesamt 3 bis 20 Kohlenstoffatomen oder unsubstituiertes oder mit C₁-C₁₂-Alkyl und/oder C₁-C₁₂-Alkoxy substituiertes Aryl mit insgesamt 6 bis 20 Kohlenstoffatomen steht,
bei dem man wenigstens ein 2-substituiertes 4-Hydroxy-4-methyl-tetrahydropyran der allgemeinen Formel (II.1) bereitstellt 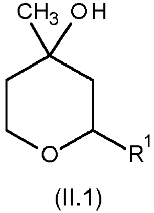
wobei R¹ die zuvor angegebenen Bedeutungen hat und die Verbindung der allgemeinen Formel (11.1) einer Umsetzung mit dem Keten (III.1)

   CH₂=C=O (III.1)
unterzieht.

### BESCHREIBUNG DER ERFINDUNG

Sofern im Folgenden nicht genauer angegeben, bezeichnen die Begriffe
"Tetrahydropyranylester",
"4-Hydroxy-tetrahydropyranverbindung",
"2-substituiertes 4-Hydroxy-4-methyl-tetrahydropyran",
"2-substituiertes 4-Methyl-tetrahydropyranyl-4-acetat"

im Rahmen der Erfindung cis/trans-Gemische jedweder Zusammensetzung sowie die reinen Konformations-Isomere. Die zuvor genannten Begriffe bezeichnen weiterhin alle Enantiomere in Reinform sowie racemische und optisch aktive Gemische der Enantiomeren dieser Verbindungen.

Sofern im Folgenden von cis- und trans-Diastereomeren der Verbindungen (I) die Rede ist, wird jeweils nur eine der enantiomeren Formen abgebildet. Lediglich zur Veranschaulichung werden im Folgenden beispielhaft die Isomeren des 2-Isobutyl-4-methyl-tetrahydropyran-4-yl-acetats (I.1a) wiedergegeben:

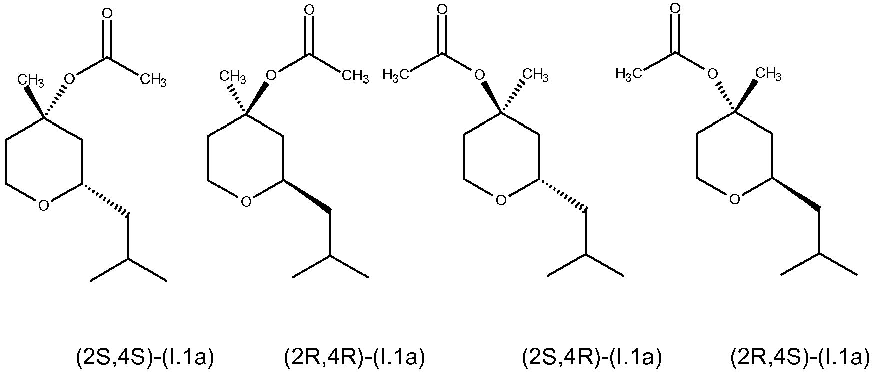

Im Rahmen der vorliegenden Erfindung steht der Ausdruck geradkettiges oder verzweigtes Alkyl vorzugsweise für C₁-C₆-Alkyl und besonders bevorzugt für C₁-C₄-Alkyl. Alkyl steht insbesondere für Methyl, Ethyl, n-Propyl, Isopropyl, n-Butyl, Isobutyl (2-Methylpropyl), sec.-Butyl (1-Methylpropyl), tert.-Butyl (1,1-Dimethylethyl), n-Pentyl oder n-Hexyl. Speziell steht Alkyl für Methyl, Ethyl, n-Propyl, Isopropyl, oder Isobutyl.

Im Rahmen der vorliegenden Erfindung steht der Ausdruck geradkettiges oder verzweigtes Alkoxy vorzugsweise für C₁-C₆-Alkoxy und besonders bevorzugt für C₁-C₄-Alkoxy. Alkoxy steht insbesondere für Methoxy, Ethoxy, n-Propyloxy, Isopropyloxy, n-Butyloxy, Isobutyloxy, sec.-Butyloxy, tert.-Butyloxy, n-Pentyloxy oder n-Hexyloxy. Speziell steht Alkoxy für Methoxy, Ethoxy, n-Propyloxy, Isopropyloxy, oder Isobutyloxy.

Im Rahmen der vorliegenden Erfindung steht der Ausdruck geradkettiges oder verzweigtes Alkenyl vorzugsweise für C₂-C₆-Alkenyl und besonders bevorzugt für C₂-C₄-Alkenyl. Der Alkenylrest weist neben Einfachbindungen noch eine oder mehrere, bevorzugt 1 bis 3, besonders bevorzugt 1 oder 2 und ganz besonders bevorzugt eine ethylenische Doppelbindung auf. Alkenyl steht insbesondere für Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl oder 2-Methyl-2-propenyl.

Im Rahmen der Erfindung bezeichnet Cycloalkyl einen cycloaliphatischen Rest mit vorzugsweise 3 bis 10, besonders bevorzugt 5 bis 8, Kohlenstoffatomen. Beispiele für Cycloalkylgruppen sind insbesondere Cyclopropyl, Cyclobutyl, Cyclopentyl, Cyclohexyl, Cycloheptyl oder Cyclooctyl. Speziell steht Cycloalkyl für Cyclohexyl.

Substituierte Cycloalkylgruppen können in Abhängigkeit von der Ringgröße einen oder mehrere (z. B. 1, 2, 3, 4 oder 5) Substituenten aufweisen. Diese sind vorzugsweise unabhängig voneinander ausgewählt unter C₁-C₆-Alkyl und C₁-C₆-Alkoxy. Die Cycloalkylgruppen tragen im Falle einer Substitution vorzugsweise eine oder mehrere, beispielsweise eine, zwei, drei, vier oder fünf C₁-C₆-Alkylgruppen. Beispiele für substituierte Cycloalkylgruppen sind insbesondere 2- und 3-Methylcyclopentyl, 2- und 3-Ethylcyclopentyl, 2-, 3- und 4-Methylcyclohexyl, 2-, 3- und 4-Ethylcyclohexyl, 2-, 3- und 4-Propylcyclohexyl, 2-, 3- und 4-Isopropylcyclohexyl, 2-, 3- und 4-Butylcyclohexyl und 2-, 3- und 4-Isobutylcyclohexyl.

Der Ausdruck "Aryl" umfasst im Rahmen der vorliegenden Erfindung ein- oder mehrkernige aromatische Kohlenwasserstoffreste mit üblicherweise 6 bis 14, besonders bevorzugt 6 bis 10 Kohlenstoffatomen. Beispiele für Aryl sind insbesondere Phenyl, Naphthyl, Indenyl, Fluorenyl, Anthracenyl, Phenanthrenyl, Naphthacenyl, Chrysenyl, Pyrenyl, etc. und speziell Phenyl oder Naphthyl.

Substituierte Aryle können in Abhängigkeit von der Anzahl und Größe ihrer Ringsysteme einen oder mehrere (z. B. 1, 2, 3, 4 oder 5) Substituenten aufweisen. Diese sind vorzugsweise unabhängig voneinander ausgewählt unter C₁-C₆-Alkyl und C₁-C₆-Alkoxy. Beispiele für substituierte Arylreste sind 2-, 3- und 4-Methylphenyl, 2,4-, 2,5-, 3,5- und 2,6-Dimethylphenyl, 2,4,6-Trimethylphenyl, 2-, 3- und 4-Ethylphenyl, 2,4-, 2,5-, 3,5- und 2,6-Diethylphenyl, 2,4,6-Triethylphenyl, 2-, 3- und 4-Propylphenyl, 2,4-, 2,5-, 3,5- und 2,6-Dipropylphenyl, 2,4,6-Tripropylphenyl, 2-, 3- und 4-Isopropylphenyl, 2,4-, 2,5-, 3,5- und 2,6-Diisopropylphenyl, 2,4,6-Triisopropylphenyl, 2-, 3- und 4-Butylphenyl, 2,4-, 2,5-, 3,5- und 2,6-Dibutylphenyl, 2,4,6-Tributylphenyl, 2-, 3- und 4-Isobutylphenyl, 2,4-, 2,5-, 3,5- und 2,6-Diisobutylphenyl, 2,4,6-Triisobutylphenyl, 2-, 3- und 4-sec-Butylphenyl, 2,4-, 2,5-, 3,5- und 2,6-Di-sec-butylphenyl, 2,4,6-Tri-sec-butylphenyl, 2-, 3- und 4-tert.-Butylphenyl, 2,4-, 2,5-, 3,5- und 2,6-Di-tert.-butylphenyl und 2,4,6-Tri-tert.-butylphenyl.

Die im Folgenden angegebenen geeigneten und bevorzugten Bedingungen für die Umsetzung einer Verbindung der allgemeinen Formel (II) mit einem Keten (III) gelten gleichermaßen für die Umsetzung einer Verbindung der allgemeinen Formel (II.1) mit dem Keten (III.1), sofern nichts anderes angegeben ist.

Bevorzugt steht R¹ in den Verbindungen der Formel (I), (II), (I.1) und (II.1) für geradkettiges oder verzweigtes C₁-C₁₂-Alkyl oder geradkettiges oder verzweigtes C₂-C₁₂-Alkenyl. Besonders bevorzugt steht R¹ für geradkettiges oder verzweigtes C₁-C₆-Alkyl oder geradkettiges oder verzweigtes C₂-C₆-Alkenyl. In einer weiteren bevorzugten Ausführung steht R¹ für Phenyl.

Bevorzugte Bedeutungen für den Rest R¹ sind somit Methyl, Ethyl, n-Propyl, Isopropyl, n-Butyl, Isobutyl, n-Pentyl, n-Hexyl oder Phenyl.

Besonders bevorzugt steht R¹ für n-Propyl oder Isobutyl (2-Methylpropyl).

Bevorzugt stehen R², R³ und R⁴ alle für Wasserstoff.

Bevorzugt steht R⁵ für Methyl oder Ethyl, besonders bevorzugt Methyl.

R^{a} und R^{b} stehen für Wasserstoff.

Für den Einsatz in dem erfindungsgemäßen Verfahren geeignete 4-Hydroxy-tetrahydropyranverbindungen der allgemeinen Formel (II) und Verfahren zu deren Herstellung sind dem Fachmann prinzipiell bekannt.

In einer speziellen Ausführungsform wird in dem erfindungsgemäßen Verfahren ein 2-substituiertes 4-Hydroxy-4-methyl-tetrahydropyran der allgemeinen Formel (11.1) eingesetzt 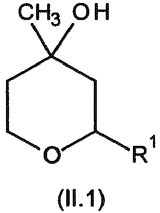 wobei R¹ die zuvor angegebene Bedeutung hat.

Vorzugsweise wird zur Bereitstellung des 2-substituierten 4-Hydroxy-4-methyl-tetra-hydropyrans der allgemeinen Formel (11.1)
a) 3-Methylbut-3-en-1-ol der Formel (IV) 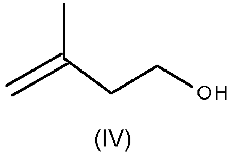 mit einem Aldehyd der Formel (V)

   R¹-CHO (V)

   worin
   R¹ für geradkettiges oder verzweigtes C₁-C₁₂-Alkyl, geradkettiges oder verzweigtes C₂-C₁₂-Alkenyl, unsubstituiertes oder mit C₁-C₁₂-Alkyl und/oder C₁-C₁₂-Alkoxy substituiertes Cycloalkyl mit insgesamt 3 bis 20 Kohlenstoffatomen oder unsubstituiertes oder mit C₁-C₁₂-Alkyl und/oder C₁-C₁₂-Alkoxy substituiertes Aryl mit insgesamt 6 bis 20 Kohlenstoffatomen steht,
   in Gegenwart eines sauren Katalysators umgesetzt, wobei ein Reaktionsgemisch erhalten wird, das wenigstens ein 2-substituiertes 4-Hydroxy-4-methyl-tetrahydropyran der allgemeinen Formel (II.1) enthält, worin R¹ die zuvor angegebene Bedeutung hat,
b) gegebenenfalls das Reaktionsgemisch aus Schritt a) einer Auftrennung unter Erhalt wenigstens einer an den 2-substituierten 4-Hydroxy-4-methyl-tetrahydropyranen der allgemeinen Formel (II.1) angereicherten Fraktion unterzogen wird.

Derartige Verfahren sind z. B. in der EP 1 493 737 A1, WO 2011/147919, WO 2010/133473, WO 2011/154330 und PCT/EP2013/071409 beschrieben, worauf hier in vollem Umfang Bezug genommen wird.

In einer speziellen Ausführungsform wird das Reaktionsgemisch aus Schritt a) einer Auftrennung unter Erhalt wenigstens einer an den 2-substituierten 4-Hydroxy-4-methyl-tetrahydropyranen der allgemeinen Formel (I.1) angereicherten Fraktion und einer an den 2-substituierten 4-Hydroxy-4-methyl-tetrahydropyranen der allgemeinen Formel (I.1) abgereicherten Fraktion unterzogen (= Schritt b)).

Einer der Ausgangsstoffe für Schritt a) des erfindungsgemäßen Verfahrens ist 3-Methylbut-3-en-1-ol (Isoprenol) der Formel (IV),

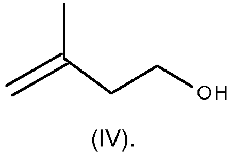

Isoprenol ist nach bekannten Verfahren aus Isobuten und Formaldehyd in jedem Maßstab gut zugänglich und kommerziell verfügbar. An die Reinheit, Qualität oder Herstellverfahren des erfindungsgemäß einzusetzenden Isoprenols sind keine besonderen Anforderungen zu stellen. Es kann in handelsüblicher Qualität und Reinheit in Schritt a) des erfindungsgemäßen Verfahrens eingesetzt werden. Bevorzugt setzt man Isoprenol ein, das eine Reinheit von 90 Gew.-% oder darüber hat, besonders bevorzugt solches mit einer Reinheit von 95 bis 100 Gew.-% und ganz besonders bevorzugt solches mit einer Reinheit von 97 bis 99,9 Gew.-% oder noch mehr bevorzugt 98 bis 99,8 Gew.-%.

Ein weiterer Ausgangsstoff für Schritt a) des erfindungsgemäßen Verfahrens ist ein Aldehyd der Formel (V) R¹-CHO, wobei R¹ in der Formel (V) die zuvor angegebene Bedeutung hat.

Bevorzugt einzusetzende Aldehyde der Formel (V) sind: Acetaldehyd, Propionaldehyd, Butyraldehyd, Valeraldehyd, Isovaleraldehyd, Hexanal, Heptanal, Benzaldehyd, Citral, Citronellal. Erfindungsgemäß ganz besonders bevorzugt einzusetzende Aldehyde der Formel (V) sind Butyraldehyd, Isovaleraldehyd und Benzaldehyd, insbesondere Butyraldehyd und Isovaleraldehyd.

Bevorzugt werden in Schritt a) das 3-Methylbut-3-en-ol (IV) und der Aldehyd (V) in einem molaren Verhältnis von etwa 1 zu 2 bis 2 zu 1, besonders bevorzugt von 0,7 zu 1 bis 2 zu 1, insbesondere von 1 zu 1 bis 2 zu 1, eingesetzt. In einer speziellen Ausführung werden in Schritt a) das 3-Methylbut-3-en-ol (IV) und der Aldehyd (V) in einem molaren Verhältnis von 1 zu 1 bis 1,5 zu 1 eingesetzt.

Bevorzugt erfolgt die Umsetzung in Schritt a) in Gegenwart eines sauren Katalysators. Prinzipiell kann für die Umsetzung in Schritt a) jeder saure Katalysator verwendet werden, d. h. jede Substanz, die Brönstedt- oder Lewis-Acidität aufweist. Beispiele für geeignete Katalysatoren sind Protonensäuren, wie Salzsäure, Schwefelsäure, Phosphorsäure, Methansulfonsäure und p-Toluolsulfonsäure, saure molekulare Elementverbindungen, wie Aluminiumchlorid, Bortrifluorid, Zinkchlorid, Zinntetrachlorid und Titantetrachlorid; oxidische saure Festkörper wie Zeolithe, Silikate, Aluminate, Alumosilikate, Tone und stark saure lonentauscher.

Unter dem Begriff stark saurer Kationenaustauscher wird dabei ein Kationenaustauscher in der H⁺-Form verstanden, der stark saure Gruppen aufweist. Bei den stark sauren Gruppen handelt es sich in der Regel um Sulfonsäuregruppen. Die sauren Gruppen sind in der Regel angebunden an eine Polymermatrix, die z. B. gelförmig bzw. makroporös sein kann. Eine bevorzugte Ausführungsform des erfindungsgemäßen Verfahrens ist dementsprechend dadurch gekennzeichnet, dass man einen stark sauren, Sulfonsäuregruppen aufweisenden Kationenaustauscher einsetzt. Geeignete stark saure Kationenaustauscher sind in der WO 2010/133473 und WO 2011/154330 beschrieben, worauf hier in vollem Umfang Bezug genommen wird.

Die Umsetzung in Schritt a) kann wahlweise auch zusätzlich in Gegenwart eines unter den Reaktionsbedingungen inerten externen organischen Lösungsmittels durchgeführt werden. Geeignete Lösungsmittel sind beispielsweise tert-Butylmethylether, Cyclohexan, Dekalin, Hexan, Heptan, Ligroin, Petrolether, Toluol oder Xylol. Die genannten Lösungsmittel können alleine oder in Form von Gemischen untereinander eingesetzt werden. Bevorzugt führt man die Umsetzung in Schritt a) ohne Zusatz eines externen organischen Lösungsmittels durch.

Bevorzugt wird in Schritt b) das Reaktionsgemisch aus Schritt a) einer destillativen Auftrennung unter Erhalt wenigstens einer an den 2-substituierten 4-Hydroxy-4-methyl-tetrahydropyranen der allgemeinen Formel (II.1) angereicherten Fraktion und einer an den 2-substituierten 4-Hydroxy-4-methyl-tetrahydropyranen der allgemeinen Formel (II.1) abgereicherten Fraktion unterzogen. Geeignete Vorrichtungen zur destillativen Auftrennung umfassen Destillationskolonnen, wie Bodenkolonnen, die mit Glocken, Siebplatten, Siebböden, Packungen, Füllkörpern, Ventilen, Seitenabzügen, etc. ausgerüstet sein können, Verdampfer, wie Dünnschichtverdampfer, Fallfilmverdampfer, Zwangsumlaufverdampfer, Sambay-Verdampfer (agitated thin-film evaporator), etc. und Kombinationen davon. Die Destillationskolonnen können trennwirksame Einbauten aufweisen, die vorzugsweise ausgewählt sind unter Trennböden, geordnete Packungen, z. B. Blech- oder Gewebepackungen, wie Sulzer Mellapak®, Sulzer BX, Montz B1 oder Montz A3 oder Kühni Rombopak, oder regellose Schüttungen von Füllkörpern, wie z. B. Dixon-Ringen, Raschig-Ringen, High-Flow-Ringen oder Raschig-Super-Ringen. Ein bevorzugtes Verfahren zur Herstellung und Isolierung von 2-sustituierten 4-Hydroxy-4-methyl-tetrahydropyranolen durch Umsetzung von 3-Methylbut-3-en-1-ol (Isoprenol) mit den entsprechenden Aldehyden in Gegenwart eines stark sauren Kationenaustauschers und anschließender Isolierung bzw. destillativer Abtrennung in einer Trennwandkolonne oder in einer Zusammenschaltung von zwei Destillationskolonnen in Form einer thermischen Kopplung ist in der WO 2011/154330 beschrieben. Auf die Offenbarung dieses Dokuments wird hier Bezug genommen.

Erfindungsgemäß wird in dem erfindungsgemäßen Verfahren wenigstens eine 4-Hydroxy-tetrahydropyranverbindung der allgemeinen Formel (II) bzw. (II.1) mit einem Keten der Formel (III) bzw. (III.1) umgesetzt.

Für den Einsatz in dem erfindungsgemäßen Verfahren eignen sich ganz allgemein Ketenverbindungen der allgemeinen Formel (III) CR^{a}R^{b}=C=O, wobei R^{a} und R^{b} für Wasserstoff stehen.

Der einfachste Vertreter der Ketenverbindungen ist das Keten der Formel (III.1) CH₂=C=O (Ethenon). Dieses wird erfindungsgemäß eingesetzt.

Bevorzugt erzeugt man das Keten (III.1) durch Hochtemperatur-Pyrolyse von Aceton oder Essigsäure bei Temperaturen, die in der Regel höher als 650 °C sind. Bevorzugt liegt die Temperatur zur Erzeugung des Ketens (III.1) in einem Bereich von 650 bis 1000 °C, besonders bevorzugt von 700 bis 900 °C.

In einer speziellen Ausführungsform wird das Keten (III.1) unter vermindertem Druck hergestellt. Bevorzugt liegt der Druck in einem Bereich von etwa 100 bis 900 mbar, besonders bevorzugt von 300 bis 500 mbar, insbesondere von 350 bis 450 mbar. In einer alternativen Ausführungsform wird das Keten (III.1) unter Umgebungsdruck ("drucklos") hergestellt. Bevorzugt liegt der Druck dann in einem Bereich von etwa 950 bis 1050 mbar.

Da Ketenverbindungen (III) beziehungsweise das Keten (III.1) äußerst reaktive Verbindungen sind, die stark zur Dimerisierung unter Bildung von Diketenen neigen, verwendet man in dem erfindungsgemäßen Verfahren vorzugsweise eine Ketenverbindung, welche erst kurz vorher hergestellt worden ist. Das erfindungsgemäße Verfahren gestaltet sich besonders vorteilhaft, wenn man Keten (III.1) verwendet, welches unmittelbar vor der Umsetzung in dem erfindungsgemäßen Verfahren hergestellt worden ist, z. B. durch thermische Spaltung von Aceton, Essigsäure oder Essigsäureanhydrid oder durch Dehydrochlorierung von Acetylchlorid mit Basen, wie Triethylamin.

In einer ersten Variante des erfindungsgemäßen Verfahrens wird das Keten (III.1) unter der Flüssigkeitsoberfläche in das Reaktionsgemisch eingeführt, so dass es das Reaktionsgemisch durchperlt. Vorteilhafterweise wird das Keten unter intensivem Rühren in das Reaktionsgemisch geführt, so dass im Wesentlichen kein Keten in größeren Mengen in die Gasphase übertritt. Der Druck des Ketens (III.1) muss ausreichend hoch sein, um den hydrostatischen Druck des Reaktionsgemisches oberhalb der Keten-Einspeisung zu überwinden, gegebenenfalls unterstützt durch einen Inertgasstrom, z. B. Stickstoff.

Das Keten (III.1) kann über beliebige geeignete Vorrichtungen eingespeist werden. Wichtig sind dabei eine gute Verteilung und eine schnelle Durchmischung. Es eignen sich z. B. Begasungslanzen, die fest installiert sein können, oder vorzugsweise Düsen. Die Düsen können am oder in der Nähe des Reaktorbodens vorgesehen sein. Die Düsen können hierzu als Öffnungen einer den Reaktor umgebenden Hohlkammer ausgebildet sein. Bevorzugt werden jedoch Tauchdüsen mit geeigneten Zuleitungen eingesetzt. Mehrere Düsen können z. B. in Form eines Kranzes angeordnet sein. Die Düsen können nach oben oder nach unten weisen. Die Düsen weisen vorzugsweise schräg nach unten.

In einer zweiten Variante des erfindungsgemäßen Verfahrens wird das Keten (III.1) unter vermindertem Druck hergestellt und unter vermindertem Druck mit wenigstens einer 4-Hydroxy-tetrahydropyranverbindung der allgemeinen Formel (II) umgesetzt. Bevorzugt liegt der Druck bei der Herstellung und der Umsetzung des Ketens (III.1) in einem Bereich von etwa 100 bis 900 mbar, besonders bevorzugt von 300 bis 500 mbar, insbesondere von 350 bis 450 mbar.

Verfahren und Vorrichtungen zur Herstellung von Ethenon sind z. B. in Organic Syntheses, Coll. Vol. 1, S. 330 (1941) und Vol. 4, S. 39 (1925) sowie in der Chemiker Zeitung 97, Nr. 2, Seiten 67 bis 73 (1979) beschrieben. Soll in dem erfindungsgemäßen Verfahren eine Ketenverbindung CR^{a}R^{b}=C=O (III) eingesetzt werden, wobei R^{a} und R^{b} von Wasserstoff verschiedene Bedeutungen besitzen, so kann die Herstellung nach prinzipiell bekannten Verfahren erfolgen. Dazu zählt z. B. die Eliminierung von Halogenwasserstoff aus Carbonsäurehalogeniden mit nachbarständigem Wasserstoff. Derartige Verfahren sind z. B. in Organikum, VEB Deutscher Verlag der Wissenschaften, 16. Auflage, Berlin 1986, Kapitel 3.1.5, speziell Seite 234 beschrieben. Die Herstellung von Ketenverbindungen gelingt auch durch Arndt-Eistert-Synthese durch Umsetzung eines Carbonsäurehalogenids mit Diazomethan.

Ein Überschuss an der Ketenverbindung (III) (bzw. (III.1)) kann zu unerwünschten Nebenreaktionen führen. Daher erfolgt die Umsetzung der Verbindung der allgemeinen Formel (II) mit dem Keten (III) vorzugsweise unter Verwendung von höchstens äquimolaren Mengen an der Ketenverbindung (III). Bevorzugt ist ein geringer molarer Überschuss an der Verbindung der allgemeinen Formel (II).

Vorzugsweise erfolgt die Umsetzung der 4-Hydroxy-tetrahydropyranverbindung der allgemeinen Formel (II) mit der Ketenverbindung (III) beziehungsweise dem Keten (III.1) so, dass eine Akkumulation der Ketenverbindung im Reaktionsgemisch zu jedem Zeitpunkt der Umsetzung vermieden wird.

Bevorzugt erfolgt die Umsetzung der Verbindung der allgemeinen Formel (II) mit dem Keten (III) so, dass so lange Keten in das Reaktionsgemisch eingeleitet wird, bis die Verbindung (II) im Wesentlichen vollständig umgesetzt ist. Unter "im Wesentlichen umgesetzt" wird dabei ein Umsatz von wenigstes 98 %, bevorzugt von wenigstens 99 %, verstanden.

Bevorzugt wird die Verbindung der allgemeinen Formel (II) einer Umsetzung mit einem Keten (III) bei einer Temperatur im Bereich von 0 bis 150 °C, bevorzugt von 10 bis 120 °C, unterzogen.

In einer ersten bevorzugten Ausführungsform wird die Verbindung der allgemeinen Formel (II) (bzw. (II.1)) einer Umsetzung mit einem Keten (III) (bzw. (III.1)) in Abwesenheit eines zugesetzten Katalysators unterzogen.

In einer zweiten bevorzugten Ausführungsform wird die Verbindung der allgemeinen Formel (II) (bzw. (II.1)) einer Umsetzung mit einem Keten (III) (bzw. (III.1)) in Gegenwart eines Katalysators unterzogen. Bevorzugt wird wenigstens ein Zinksalz als Katalysator eingesetzt, das auch als Hydrat bzw. Mehrfach-Hydrat vorliegen kann.

Besonders bevorzugt wird als Katalysator ein Zinksalz einer Carbonsäure, speziell einer Monocarbonsäure mit 1 bis 18 C-Atomen oder Dicarbonsäure mit 2 bis 18 C-Atomen, eingesetzt. Dazu zählen z. B. Zinkformiat, Zinkacetat, Zinkpropionat, Zinkbutyrat, Zinkstearat, Zinksuccinat oder Zinkoxalat. Besonders bevorzugt ist Zinkacetat.

Sehr vorteilhaft an dem erfindungsgemäßen Verfahren ist, dass man die Katalysatoren in der Regel nur in sehr kleinen Mengen einsetzen muss, was das Verfahren kostengünstiger macht und die Aufarbeitung des Reaktionsgemisches erleichtert. Dies gilt insbesondere für den Einsatz eines Zinksalzes als Katalysator.

Bevorzugt verwendet man den Katalysator in einer Menge von 0,01 bis 2 Gew.-%, besonders bevorzugt von 0,02 bis 0,5 Gew.-%, bezogen auf die Gesamtmenge der Verbindung (II) (bzw. (II.1)).

Zur Durchführung der erfindungsgemäßen Umsetzung geht man mit Vorteil so vor, dass man diese Umsetzung in einem geeigneten Reaktionsgefäß durchführt, welches als wesentliche Bestandteile eine gute Rühr- und/oder Mischeinrichtung, eine Dosiervorrichtung für Keten, eine Heizvorrichtung zum Starten der Reaktion und zum Aufrechterhalten der Reaktionstemperatur während der Nachreaktion, eine Kühleinrichtung zum Abführen der Reaktionswärme der exothermen Umsetzung sowie eine Vakuumpumpe enthält.

Für eine optimale Reaktionsführung ist es vorteilhaft, dass man das Keten so zudosiert, dass es im Reaktionsgemisch nie im Überschuss vorliegt, und dass das Reaktionsgemisch immer gut durchmischt wird.

Für eine optimale Reaktionsführung ist es weiterhin vorteilhaft, dass man zu schnelles Zudosieren von Keten vermeidet sowie das Ende der Umsetzung eindeutig feststellt.

Der Nachweis von Keten gelingt beispielsweise IR-spektroskopisch über die charakteristische Carbonylschwingung.

Mit Hilfe des erfindungsgemäßen Verfahrens ist es möglich, die Verbindungen der allgemeinen Formel (I) auf technisch einfache Weise in hohen Reinheiten und dennoch in ausgezeichneten Ausbeuten und Raum-Zeit-Ausbeuten herzustellen. Da die Edukte im Wesentlichen vollständig zu Produkten umgesetzt werden, zeichnet sich das erfindungsgemäße Verfahren durch eine maximale Atomökonomie aus.

Die nach dem erfindungsgemäßen Verfahren erhältlichen Zusammensetzungen eignen sich besonders vorteilhaft als Riechstoff oder zur Bereitstellung eines Riechstoffs.

Die erfindungsgemäßen Zusammensetzungen können für den Einsatz als Riechstoff mit wenigstens einem auf diesem Anwendungsgebiet üblichen Lösemittel beliebig verdünnt werden. Beispielhaft seien als geeignete Lösemittel genannt: Ethanol, Dipropylenglycol oder dessen Ether, Phthalate, Propylenglykole oder Carbonate von Diolen, bevorzugt Ethanol. Auch Wasser ist als Lösemittel zur Verdünnung der erfindungsgemäßen Duftstoffkompositionen geeignet und kann vorteilhaft zusammen mit geeigneten Emulgatoren eingesetzt werden.

Die nach dem erfindungsgemäßen Verfahren erhaltenen Riechstoffe weisen auf Grund der strukturellen und chemischen Ähnlichkeit der Komponenten eine hohe Stabilität und Haltbarkeit auf.

Die nach dem erfindungsgemäßen Verfahren erhaltenen Riechstoffe eigenen sich zur Einarbeitung in kosmetische Zusammensetzungen sowie Gebrauchs- und Verbrauchsgüter bzw. Mittel, wie sie im Folgenden näher beschrieben sind, wobei die Riechstoffe in die genannten Güter eingearbeitet oder auch auf solche aufgebracht sein können. Unter einer organoleptisch wirksamen Menge ist dabei, wie im Rahmen der gesamten vorliegenden Erfindung, insbesondere eine solche Menge zu verstehen, die bei sachgemäßer Anwendung ausreicht, beim Anwender bzw. Verbraucher einen Dufteindruck hervorzurufen.

Als kosmetische Zusammensetzungen sind alle üblichen kosmetischen Zusammensetzungen geeignet. Dabei handelt es sich bevorzugt um Parfum, Eau de Toilette, Deodorants, Seife, Duschgel, Badegel, Cremes, Lotions, Sonnenschutzmittel, Zusammensetzungen zur Reinigung und Pflege der Haare, wie Haarshampoo, Spülung, Haargel, Haarfestiger in Form von Flüssigkeiten oder Schäumen und weitere Reinigungs- oder Pflegemittel für die Haare, Zusammensetzungen zur dekorativen Anwendung am menschlichen Körper, wie kosmetische Stifte, zum Beispiel Lippenstifte, Lippenpflegestifte, Abdeckstifte (Concealer), Wangenrouge (Blusher), Lidschattenstifte, Lippenkonturenstifte, Augenkonturenstifte, Augenbrauenstifte, Korrekturstifte, Sonnenschutzstifte, Anti-Akne-Stifte und vergleichbare Produkte sowie Nagellacke und weitere Produkte zur Nagelpflege.

Die nach dem erfindungsgemäßen Verfahren erhaltenen Riechstoffe eignen sich speziell für einen Einsatz in Parfums, z. B. als Eau de Toilette, Duschgele, Badegele und Körperdeodorants.

Sie eignen sich weiterhin zur Aromatisierung von Verbrauchs- oder Gebrauchsgütern, in die sie eingearbeitet bzw. auf die sie aufgebracht werden und ihnen dadurch einen angenehmen frischen grünen Akzent verleihen. Beispiele für Verbrauchs- oder Gebrauchsgüter sind: Raumluftdeodorants (Air Care), Reinigungsmittel oder Pflegemittel für Textilien (speziell Waschmittel, Weichspüler), Textilbehandlungsmittel wie beispielsweise Bügelhilfsmittel, Putzmittel, Reinigungsmittel, Pflegemittel zur Behandlung von Oberflächen, beispielsweise von Möbeln, Fußböden, Kücheneinrichtungen, Glasscheiben und Fenstern sowie Bildschirmen, Bleichen, Toilettensteine, Entkalkungsmittel, Düngemittel, Baustoffe, Schimmelentferner, Desinfektionsmittel, Produkte für die Auto- bzw. Fahrzeugpflege und dergleichen mehr.

Die folgenden Beispiele dienen der Verdeutlichung der Erfindung, ohne sie in irgendeiner Weise zu beschränken.

### BEISPIELE

Gaschromatographische Analysen wurden nach der folgenden Methode durchgeführt:
Säule: DB WAX 30 m x 0,32 mm
FD 0,25 µm
Injektortemperatur: 200 °C; Detektortemperatur 250 °C
Temperaturprogramm: Anfangstemp.: 60 °C, mit 2 °C/min auf 120 °C,
   mit 20 °C/min auf 230 °C
Retentionszeiten: trans-Tetrahydro-2-isobutyl-4-methylpyranyl-4-acetat t_{R} = 15,1 min cis-Tetrahydro-2-isobutyl-4-methylpyranyl-4-acetat t_{R} = 18,8 min trans-Tetrahydro-2-isobutyl-4-methylpyran-4-ol t_{R} = 19,6 min cis-Tetrahydro-2-isobutyl-4-methylpyran-4-ol t_{R} = 21,5 min
Die Konzentrationen der erhaltenen Produkte (Gew.-%) wurden GC-analytisch mit internem Standard ermittelt.

### Beispiel 1:

### (Herstellung von Tetrahydro-2-isobutyl-4-methylpyranyl-4-acetat aus Tetrahydro-2-isobutyl-4-methylpyran-4-ol durch Umsetzung mit Keten)

127,08 g Tetrahydro-2-isobutyl-4-methylpyran-4-ol (0,74 Mol; Zusammensetzung vgl. Tabelle 1, Probe nach 0 h) wurden bei 90 °C vorgelegt. Keten, das durch die Pyrolyse von Aceton (0,411 mL/min) bei 700 °C erhalten wurde, wurde nach Abkühlen bei 90 °C unterhalb der Flüssigkeitsoberfläche und unter kräftigem Rühren eingeleitet. Der Umsatz der Pyrolyse lag bei ca. 48 % (bezogen auf isoliertes, nicht umgesetztes Aceton) und der Ketengehalt im Pyrolysegas lag bei 23 bis 24 %. Nach 7 h Reaktionszeit wurde die Keten-Einleitung unterbrochen und am Folgetag fortgesetzt. Nach insgesamt 10 h Reaktionszeit war das Startmaterial umgesetzt, und der Versuch wurde beendet. Insgesamt wurden während des Versuchs 92,6 g Aceton (1,59 Mol, 2,15 Äq.; bezogen auf isoliertes, nicht umgesetztes Aceton) in der Pyrolyse umgesetzt. Die Zusammensetzung der Proben ist in Tabelle 1 angegeben. Die Ausbeute an Tetrahydro-2-isobutyl-4-methylpyranyl-4-acetat lag bei 89 %.

Cis- und trans-Tetrahydro-2-isobutyl-4-methylpyranyl-4-acetatwurden per NMR-Spektroskopie charakterisiert:
Cis-Tetrahydro-2-isobutyl-4-methylpyranyl-4-acetat: ¹³C NMR (125 MHz, CDCl₃): δ = 21.7, 22.3, 22.5, 23.2, 24.3, 37.7, 43.8, 45.4, 64.6, 72.7, 80.0, 170.3 ppm.

Trans-Tetrahydro-2-isobutyl-4-methylpyranyl-4-acetat: ¹³C NMR (125 MHz, CDCl₃): δ = 22.37, 22.39, 23.2, 24.3, 26.2, 36.3, 42.5, 45.1, 63.4, 70.9, 79.3, 170.4 ppm.

**Tabelle 1: Zusammensetzung des Reaktionsgemisches**

| Reaktionszeit | trans-Tetrahydro-2-isobutyl-4-methylpyranyl-4-acetat | cis-Tetrahydro-2-isobutyl-4-methylpyranyl-4-acetat | Summe Tetrahydro-2-isobutyl-4-methylpyranyl-4-acetat | trans-Tetrahydro-2-isobutyl-4-methylpyran-4-ol | cis-Tetrahydro-2-isobutyl-4-methylpyran-4-ol | Summe Tetrahydro-2-isobutyl-4-methylpyran-4-ol |
|---|---|---|---|---|---|---|
| [h] | [%]* | [%]* | [%]* | [%]* | [%]* | [%]* |
| 0 | 0 | 0 | 0 | 22,8 | 77,1 | 99,9 |
| 2 | 3,1 | 16,0 | 19,1 | 16,9 | 52,9 | 69,8 |
| 4 | 7,4 | 35,0 | 42,4 | 15,6 | 34,5 | 50,1 |
| 6 | 11,6 | 49,8 | 61,4 | 13,7 | 18,9 | 32,6 |
| 7 | 15,2 | 60,7 | 75,9 | 10,6 | 8,4 | 19,0 |
| 8 | 18,6 | 73,4 | 92,0 | 1,5 | 2,4 | 3,9 |
| 9 | 20,6 | 74,4 | 95,0 | 0 | 0,4 | 0,4 |
| 10 | 21,2 | 73,9 | 95,1 | 0 | 0 | 0 |

| | | | | | | |
|---|---|---|---|---|---|---|
| * GC-Gew.-% | | | | | | |

### Vergleichsbeispiel 1

Tetrahydro-2-isobutyl-4-methylpyran-4-ol (5.0 g, 29 mMol, 1.0 Äq.), 4-(Dimethylamino)-pyridin (35 mg, 0.3 mMol, 0.01 Äq.) und Triethylamin (9.69 g, 96 mMol, 3.3 Äq.) wurden in Toluol (44 g) vorgelegt und unter Rühren auf 90 °C erwärmt. Dann wurde Acetylchlorid (7.52 g, 96 mMol, 3.3 Äq.) innerhalb von 2 h zugetropft. Nach insgesamt 4 h wurde auf 30 °C abgekühlt und die Reaktion durch Zugabe von Wasser (25 g) gestoppt. Die Ausbeute an Tetrahydro-2-isobutyl-4-methylpyranyl-4-acetat lag nach GC-Analytik bei 32 %.

### Vergleichsbeispiel 2

Tetrahydro-2-isobutyl-4-methylpyran-4-ol (5.0 g, 29 mMol, 1.0 Äq.), 4-(Dimethylamino)-pyridin (35 mg, 0.3 mMol, 0.01 Äq.) und Triethylamin (9.69 g, 96 mMol, 3.3 Äq.) wurden ohne Lösungsmittel unter Rühren bei 90 °C vorgelegt. Dann wurde vorsichtig Acetylchlorid (7.52 g, 96 mMol, 3.3Äq.) zugetropft, wobei unter heftiger Reaktion Pyranylacetat gebildet wurde und das Reaktionsgemisch stark heterogen wurde. Nach vollständiger Zugabe wurde noch 60 min bei 90 °C weiter gerührt, dann auf 30 °C abgekühlt, der Ansatz vorsichtig mit Wasser (50 g) versetzt und mit Toluol (30 g) extrahiert. Die Ausbeute an Tetrahydro-2-isobutyl-4-methylpyranyl-4-acetat lag nach GC-Analytik bei 26 %.

## Patentansprüche

1. Verfahren zur Herstellung von Tetrahydropyranylestern der allgemeinen Formel (I) 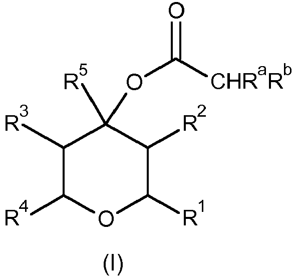 worin
R¹, R², R³ und R⁴ unabhängig voneinander für Wasserstoff, geradkettiges oder verzweigtes C₁-C₁₂-Alkyl, geradkettiges oder verzweigtes C₂-C₁₂-Alkenyl, unsubstituiertes oder mit C₁-C₁₂-Alkyl und/oder C₁-C₁₂-Alkoxy substituiertes Cycloalkyl mit insgesamt 3 bis 20 Kohlenstoffatomen oder unsubstituiertes oder mit C₁-C₁₂-Alkyl und/oder C₁-C₁₂-Alkoxy substituiertes Aryl mit insgesamt 6 bis 20 Kohlenstoffatomen stehen,
R⁵ für Wasserstoff oder geradkettiges oder verzweigtes C₁-C₁₂-Alkyl steht, und
R^{a} und R^{b} für Wasserstoff stehen,
bei dem man wenigstes eine 4-Hydroxy-tetrahydropyranverbindung der allgemeinen Formel (II) bereitstellt 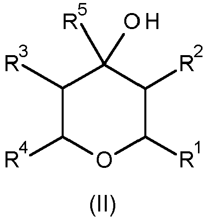 wobei R¹, R², R³, R⁴ und R⁵ die zuvor angegebenen Bedeutungen haben, und die Verbindung der allgemeinen Formel (II) einer Umsetzung mit einem Keten (III)
CR^{a}R^{b}=C=O (III)
unterzieht, wobei R^{a} und R^{b} die zuvor angegebenen Bedeutungen haben.

2. Verfahren nach Anspruch 1, wobei R¹ für geradkettiges oder verzweigtes C₁-C₆-Alkyl, geradkettiges oder verzweigtes C₂-C₆-Alkenyl oder Phenyl steht.

3. Verfahren nach Anspruch 1 oder 2, wobei R¹ für Methyl, Ethyl, n-Propyl, Isopropyl, n-Butyl, Isobutyl, n-Pentyl, n-Hexyl oder Phenyl, bevorzugt für n-Propyl oder Isobutyl steht.

4. Verfahren nach einem der vorhergehenden Ansprüche, wobei R², R³ und R⁴ alle für Wasserstoff stehen.

5. Verfahren nach einem der vorhergehenden Ansprüche, wobei R⁵ für Methyl oder Ethyl, bevorzugt Methyl, steht.

6. Verfahren nach einem der vorhergehenden Ansprüche, wobei die Verbindung der allgemeinen Formel (II) einer Umsetzung mit einem Keten (III) bei einer Temperatur im Bereich von 0 bis 150 °C, bevorzugt von 10 bis 120 °C, unterzogen wird.

7. Verfahren nach einem der Ansprüche 1 bis 6, wobei die Verbindung der allgemeinen Formel (II) einer Umsetzung mit einem Keten (III) in Abwesenheit eines zugesetzten Katalysators unterzogen wird.

8. Verfahren nach einem der Ansprüche 1 bis 6,wobei die Verbindung der allgemeinen Formel (II) einer Umsetzung mit einem Keten (III) in Gegenwart eines Katalysators unterzogen wird, der vorzugsweise ausgewählt ist unter Zinksalzen, besonders bevorzugt Zinksalzen von Carbonsäuren, insbesondere Zinkacetat.

9. Verfahren nach Anspruch 8, wobei der Katalysator in einer Menge von 0,01 bis 2 Gew.-%, besonders bevorzugt von 0,02 bis 0,5 Gew.-%, bezogen auf die Gesamtmenge der Verbindung (II) eingesetzt wird.

10. Verfahren nach Anspruch 1, zur Herstellung von 2-substituierten 4-Methyltetrahydropyranylestern der allgemeinen Formel (I.1) 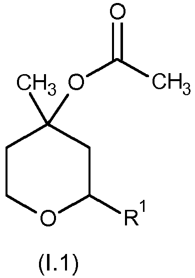 worin
R¹ für Wasserstoff, geradkettiges oder verzweigtes C₁-C₁₂-Alkyl, geradkettiges oder verzweigtes C₂-C₁₂-Alkenyl, unsubstituiertes oder mit C₁-C₁₂-Alkyl und/oder C₁-C₁₂-Alkoxy substituiertes Cycloalkyl mit insgesamt 3 bis 20 Kohlenstoffatomen oder unsubstituiertes oder mit C₁-C₁₂-Alkyl und/oder C₁-C₁₂-Alkoxy substituiertes Aryl mit insgesamt 6 bis 20 Kohlenstoffatomen steht,
bei dem man wenigstens ein 2-substituiertes 4-Hydroxy-4-methyl-tetrahydropyran der allgemeinen Formel (II.1) bereitstellt 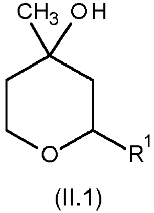
wobei R¹ die zuvor angegebenen Bedeutungen hat und die Verbindung der allgemeinen Formel (II.1) einer Umsetzung mit dem Keten (III.1)
CH₂=C=O (III.1)
unterzieht.

11. Verfahren nach Anspruch 10, wobei man zur Bereitstellung des 2-substituierten 4-Hydroxy-4-methyl-tetrahydropyrans der allgemeinen Formel (II.1):
a) 3-Methylbut-3-en-1-ol der Formel (IV) 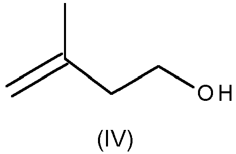 mit einem Aldehyd der Formel (V)
R¹-CHO (V)
worin
R¹ für geradkettiges oder verzweigtes C₁-C₁₂-Alkyl, geradkettiges oder verzweigtes C₂-C₁₂-Alkenyl, unsubstituiertes oder mit C₁-C₁₂-Alkyl und/oder C₁-C₁₂-Alkoxy substituiertes Cycloalkyl mit insgesamt 3 bis 20 Kohlenstoffatomen oder unsubstituiertes oder mit C₁-C₁₂-Alkyl und/oder C₁-C₁₂-Alkoxy substituiertes Aryl mit insgesamt 6 bis 20 Kohlenstoffatomen steht,
in Gegenwart eines sauren Katalysators umsetzt, wobei ein Reaktionsgemisch erhalten wird, das wenigstens ein 2-substituiertes 4-Hydroxy-4-methyl-tetrahydropyran der allgemeinen Formel (II.1) enthält, worin R¹ die zuvor angegebene Bedeutung hat,
b) gegebenenfalls das Reaktionsgemisch aus Schritt a) einer Auftrennung unter Erhalt wenigstens einer an den 2-substituierten 4-Hydroxy-4-methyl-tetrahydropyranen der allgemeinen Formel (II.1) angereicherten Fraktion unterzieht.

## Claims

1. A method for preparing tetrahydropyranyl esters of the general formula (I) 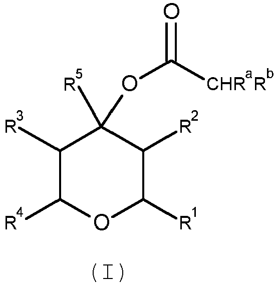 where
R¹, R², R³ and R⁴ are each independently hydrogen, straight-chain or branched C₁-C₁₂-alkyl, straight-chain or branched C₂-C₁₂-alkenyl, unsubstituted or C₁-C₁₂-alkyl- and/or C₁-C₁₂-alkoxy-substituted cycloalkyl having a total of 3 to 20 carbon atoms or unsubstituted or C₁-C₁₂-alkyl- and/or C₁-C₁₂-alkoxy-substituted aryl having a total of 6 to 20 carbon atoms,
R⁵ is hydrogen or straight-chain or branched C₁-C₁₂-alkyl, and
R^{a} and R^{b} are hydrogen,
in which at least one 4-hydroxytetrahydropyran compound of the general formula (II) is provided 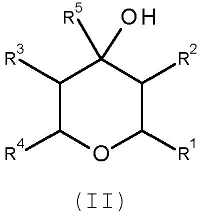
where R¹, R², R³, R⁴ and R⁵ are as defined above, and the compound of the general formula (II) is subjected to a reaction with a ketene (III),
CR^{a}R^{b}=C=O (III)
where R^{a} and R^{b} are as defined above.

2. The method according to claim 1, where R¹ is a straight-chain or branched C₁-C₆-alkyl, straight-chain or branched C₂-C₆-alkenyl or phenyl.

3. The method according to claim 1 or 2, where R¹ is methyl, ethyl, n-propyl, isopropyl, n-butyl, isobutyl, n-pentyl, n-hexyl or phenyl, preferably n-propyl or isobutyl.

4. The method according to any of the preceding claims, where R², R³ and R⁴ are all hydrogen.

5. The method according to any of the preceding claims, where R⁵ is methyl or ethyl, preferably methyl.

6. The method according to any of the preceding claims, wherein the compound of the general formula (II) is subjected to a reaction with a ketene (III) at a temperature in the range of 0 to 150°C, preferably 10 to 120°C.

7. The method according to any of claims 1 to 6, wherein the compound of the general formula (II) is subjected to a reaction with a ketene (III) in the absence of an added catalyst.

8. The method according to any of claims 1 to 6, wherein the compound of the general formula (II) is subjected to a reaction with a ketene (III) in the presence of a catalyst preferably selected from zinc salts, particularly preferably zinc salts of carboxylic acids, particularly zinc acetate.

9. The method according to claim 8, wherein the catalyst is used in an amount of 0.01 to 2% by weight, particularly preferably 0.02 to 0.5% by weight, based on the total amount of the compound (II).

10. The method according to claim 1 for preparing 2-substituted 4-methyltetrahydropyranyl esters of the general formula (I.1) 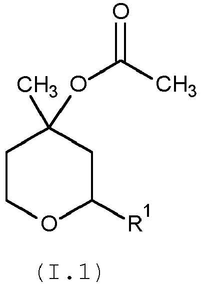 where
R¹ is hydrogen, straight-chain or branched C₁-C₁₂-alkyl, straight-chain or branched C₂-C₁₂-alkenyl, unsubstituted or C₁-C₁₂-alkyl- and/or C₁-C₁₂-alkoxy-substituted cycloalkyl having a total of 3 to 20 carbon atoms or unsubstituted or C₁-C₁₂-alkyl- and/or C₁-C₁₂-alkoxy-substituted aryl having a total of 6 to 20 carbon atoms,
in which at least one 2-substituted 4-hydroxy-4-methyltetrahydropyran of the general formula (II.1) is provided 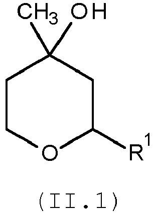
where R¹ is as defined above and the compound of the general formula (II.1) is subjected to a reaction with the ketene (III.1)
CH₂=C=O (III.1).

11. The method according to claim 10, wherein to provide the 2-substituted 4-hydroxy-4-methyltetrahydropyran of the general formula (II.1):
a) 3-methylbut-3-en-1-ol of the formula (IV) 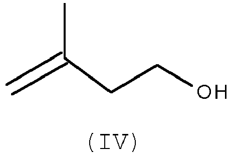 is reacted with an aldehyde of the formula (V)
R¹-CHO (V)
where
R¹ is a straight-chain or branched C₁-C₁₂-alkyl, straight-chain or branched C₂-C₁₂-alkenyl, unsubstituted or C₁-C₁₂-alkyl- and/or C₁-C₁₂-alkoxy-substituted cycloalkyl having a total of 3 to 20 carbon atoms or unsubstituted or C₁-C₁₂-alkyl- and/or C₁-C₁₂-alkoxy-substituted aryl having a total of 6 to 20 carbon atoms,
in the presence of an acidic catalyst, wherein a reaction mixture is obtained comprising at least one 2-substituted 4-hydroxy-4-methyltetrahydropyran of the general formula (II.1), where R¹ is as defined above,
b) optionally the reaction mixture from step a) is subjected to a separation to obtain at least one fraction enriched in the 2-substituted 4-hydroxy-4-methyltetrahydropyrans of the general formula (II.1) .

## Revendications

1. Procédé de fabrication d'esters de tétrahydropyranyle de la formule générale (I) : 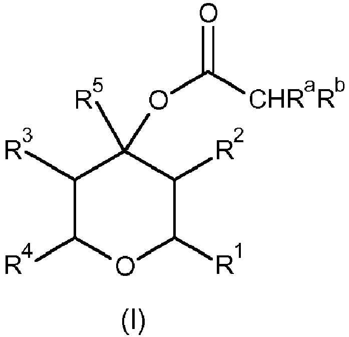 dans laquelle
R¹, R², R³ et R⁴ représentent indépendamment les uns des autres l'hydrogène, un alkyle en C₁-C₁₂ linéaire ou ramifié, un alcényle en C₂-C₁₂ linéaire ou ramifié, un cycloalkyle non substitué ou substitué avec alkyle en C₁-C₁₂ et/ou alcoxy en C₁-C₁₂, contenant au total 3 à 20 atomes de carbone, ou un aryle non substitué ou substitué avec alkyle en C₁-C₁₂ et/ou alcoxy en C₁-C₁₂, contenant au total 6 à 20 atomes de carbone,
R⁵ représente l'hydrogène ou un alkyle en C₁-C₁₂ linéaire ou ramifié, et
R^{a} et R^{b} représentent l'hydrogène,
selon lequel au moins un composé de 4-hydroxytétrahydropyrane de la formule générale (II) est préparé 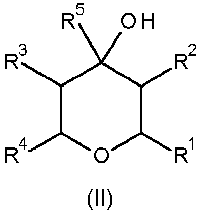
dans laquelle R¹, R², R³, R⁴ et R⁵ ont les significations indiquées précédemment, et le composé de la formule générale (II) est soumis à une réaction avec un cétène (III) :
CR^{a}R^{b}=C=O (III)
R^{a} et R^{b} ayant les significations indiquées précédemment.

2. Procédé selon la revendication 1, dans lequel R¹ représente un alkyle en C₁-C₆ linéaire ou ramifié, un alcényle en C₂-C₆ linéaire ou ramifié ou un phényle.

3. Procédé selon la revendication 1 ou 2, dans lequel R¹ représente méthyle, éthyle, n-propyle, isopropyle, n-butyle, isobutyle, n-pentyle, n-hexyle ou phényle, de préférence n-propyle ou isobutyle.

4. Procédé selon l'une quelconque des revendications précédentes, dans lequel R², R³ et R⁴ représentent tous l'hydrogène.

5. Procédé selon l'une quelconque des revendications précédentes, dans lequel R⁵ représente méthyle ou éthyle, de préférence méthyle.

6. Procédé selon l'une quelconque des revendications précédentes, dans lequel le composé de la formule générale (II) est soumis à une réaction avec un cétène (III) à une température dans la plage allant de 0 à 150 °C, de préférence de 10 à 120 °C.

7. Procédé selon l'une quelconque des revendications 1 à 6, dans lequel le composé de la formule générale (II) est soumis à une réaction avec un cétène (III) en l'absence d'un catalyseur ajouté.

8. Procédé selon l'une quelconque des revendications 1 à 6, dans lequel le composé de la formule générale (II) est soumis à une réaction avec un cétène (III) en présence d'un catalyseur, qui est de préférence choisi parmi les sels de zinc, de manière particulièrement préférée les sels de zinc d'acides carboxyliques, notamment l'acétate de zinc.

9. Procédé selon la revendication 8, dans lequel le catalyseur est utilisé en une quantité de 0,01 à 2 % en poids, de manière particulièrement préférée de 0,02 à 0,5 % en poids, par rapport à la quantité totale du composé (II).

10. Procédé selon la revendication 1, pour la fabrication d'esters de 4-méthyltétrahydropyranyle 2-substitués de la formule générale (I.1) : 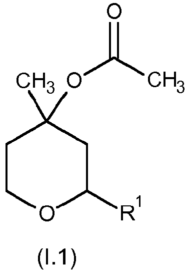 dans laquelle
R¹ représente l'hydrogène, un alkyle en C₁-C₁₂ linéaire ou ramifié, un alcényle en C₂-C₁₂ linéaire ou ramifié, un cycloalkyle non substitué ou substitué avec alkyle en C₁-C₁₂ et/ou alcoxy en C₁-C₁₂, contenant au total 3 à 20 atomes de carbone, ou un aryle non substitué ou substitué avec alkyle en C₁-C₁₂ et/ou alcoxy en C₁-C₁₂, contenant au total 6 à 20 atomes de carbone,
selon lequel au moins un 4-hydroxy-4-méthyl-tétrahydropyrane 2-substitué de la formule générale (II.1) est préparé 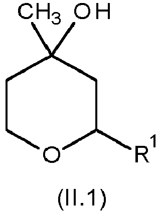
dans laquelle R¹ a les significations indiquées précédemment et le composé de la formule générale (II.1) est soumis à une réaction avec le cétène (III.1) :
CH₂=C=O (III.1).

11. Procédé selon la revendication 10, dans lequel, pour la préparation du 4-hydroxy-4-méthyl-tétrahydropyrane 2-substitué de la formule générale (II.1) :
a) du 3-méthylbut-3-én-1-ol de la formule (IV) : 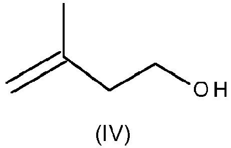 est mis en réaction avec un aldéhyde de la formule (V) :
R¹-CHO (V)
dans laquelle
R¹ représente un alkyle en C₁-C₁₂ linéaire ou ramifié, un alcényle en C₂-C₁₂ linéaire ou ramifié, un cycloalkyle non substitué ou substitué avec alkyle en C₁-C₁₂ et/ou alcoxy en C₁-C₁₂, contenant au total 3 à 20 atomes de carbone, ou un aryle non substitué ou substitué avec alkyle en C₁-C₁₂ et/ou alcoxy en C₁-C₁₂, contenant au total 6 à 20 atomes de carbone,
en présence d'un catalyseur acide, un mélange réactionnel étant obtenu, qui contient au moins un 4-hydroxy-4-méthyl-tétrahydropyrane 2-substitué de la formule générale (II.1), dans laquelle R¹ a la signification indiquée précédemment,
b) le mélange réactionnel de l'étape a) est éventuellement soumis à une séparation avec obtention d'au moins une fraction enrichie en 4-hydroxy-4-méthyl-tétrahydropyranes 2-substitués de la formule générale (II.1) .
